(19)

Europäisches
Patentamt
European
Patent Office
Office européen
des brevets

(11) **EP 2 177 499 A1**

(12) **EUROPEAN PATENT APPLICATION**

(43) Date of publication:
**21.04.2010 Bulletin 2010/16**

(51) Int Cl.:
*C07C 51/12* [(2006.01)]  *C07C 67/36* [(2006.01)]
*C07C 67/37* [(2006.01)]

(21) Application number: **08253322.5**

(22) Date of filing: **13.10.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **BP Chemicals Limited
Sunbury-on-Thames, Middlesex TW16 7BP (GB)**

(72) Inventors:
• **Law, David, John
Hull
East Yorkshire, HU12 8DS (GB)**

• **Muskett, Michael, James
Naperville, Illinois 60563 (US)**
• **Corma-Canos, Avelino
46022 Valencia (ES)**

(74) Representative: **Brooke, Caron et al
BP International Limited
Global Patents & Technology Law (Central)
Chertsey Road
Sunbury-on-Thames, Middlesex
TW16 7LN (GB)**

(54) **Carbonylation process**

(57)    A process for the production of at least one of acetic acid and methyl acetate by carbonylating at least one carbonylatable reactant selected from methanol, dimethyl ether and dimethyl carbonate with carbon monoxide in the presence of a zeolite catalyst of structure type MOR which has been dealuminated.

EP 2 177 499 A1

**Description**

This invention relates to a process for the carbonylation of a carbonylatable reactant selected from methanol, dimethyl ether and dimethyl carbonate with carbon monoxide in the presence of a dealuminated zeolite catalyst of structure type MOR.

Classically, zeolites are defined as aluminosilicates with open 3-dimensional framework structures composed of comer-sharing $TO_4$ tetrahedra, where T is aluminium or silicon. Cations that balance the charge of the anionic framework are loosely associated with the framework oxygens and the remaining pore colume is filled with water molecules. The non-framework cations are generally exchangeable and the water molecules removable. The tetrahedra are connected forming rings and cages including pores and pore networks that extend throughout the zeolite crystal. The rings of a zeolite are usually formed of 8, 10, or 12 tetrahedral units, although zeolites having larger rings have been synthesised. The framework type of a zeolite is assigned a three letter framework type code by the International Zeolite Association (IZA). For example, MOR is assigned to zeolitic materials which have the framework structure of mordenite. Descriptions of all the framework types that have been assigned codes by IZA are included in the Database of Zeolite Structures (www.iza-structure.org). Zeolite structures are also defined in the 'Atlas of Zeolite Structure Types', W M Meier, D H Olson and Ch Baerlocher, 6th revised edition, 2007, Elsevier.

Zeolites having framework type code MOR have 12-membered ring main channels with intersecting 8-membered ring channels. Examples of zeolites having the MOR framework type include mordenite.

Zeolites, in general, both natural and synthetic, have been demonstrated to have catalytic properties for various types of chemical processes. In particular, mordenite has been shown to catalyse the carbonylation of methanol and/or dimethyl ether with carbon monoxide to produce acetic acid and/or methyl acetate. For example, the carbonylation of methanol with carbon monoxide in the presence of a copper, nickel, iridium, rhodium or cobalt loaded mordenite catalyst to produce acetic acid, is described, for example in EP-A-0 596 632. In WO 2006/121778 there is described a process for the production of a lower alkyl ester of a lower aliphatic carboxylic acid by carbonylating under substantially anhydrous conditions a lower alkyl ether with carbon monoxide in the presence of a mordenite or ferrierite zeolite catalyst. In WO 2005/105720 there is described a process for the preparation of carboxylic acids and derivatives thereof in the presence of mordenite which has framework elements in addition to aluminium and silicon and which has also been loaded with copper, nickel, iridium, rhodium or cobalt.

Techniques for dealumination of zeolites are known and include hydrothermal treatment, mineral acid treatment with, for example, HCl, $HNO_3$, and $H_2SO_4$, and chemical treatment with $SiCl_4$ or EDTA.

US 3,551,353 describes a process for the dealumination of mordenite by contacting steam and mineral acid in alternate steps. The dealuminised mordenite is disclosed to be active for hydrocarbon conversion reactions such as cracking.

US 4,588,496 describes a process for the catalytic cracking of hydrocarbon feedstocks wherein the catalyst comprises supported dealuminated Y-zeolite prepared by contacting the zeolite with an aqueous solution of a fluorosilicate salt.

US 5,411,724 describes a method for removing aluminium from the framework of a zeolite by forming a mixture of the ammonium or alkaline metal form of the zeolite in the hydrated state and a crystalline ammonium fluoro-halo-metallate salt; heating the mixture at elevated temperature to form a fluoro-halo-aluminate complex salt ; and removing the formed fluoro-halo-alumniate complex salt.

It is highly desirable in carbonylation processes to improve catalyst performance. Performance measures include product selectivity, product yield, catalyst stability and lifetime.

It has now been found that the use of zeolites of structure type MOR which have been dealuminated provide improved catalyst performance in carbonylation reactions.

In particular, it has been found that, MOR zeolites in which aluminium has been at removed from its 12-member ring channels and/or from the surface of the MOR zeolite provide superior carbonylation catalyst performance compared to non-dealuminated MOR zeolites.

Accordingly, the present invention provides a process for the production of at least one of acetic acid and methyl acetate which process comprises the carbonylation of at least one carbonylatable reactant selected from methanol, dimethyl ether and dimethyl carbonate with carbon monoxide in the presence of a zeolite catalyst of structure type MOR, wherein said zeolite is dealuminated.

Zeolites of framework type MOR include mordenite.

Zeolites of structure type MOR, including mordenite, are available from commercial sources, generally in the Na, $NH_4$ form or H- form of the zeolite. The $NH_4$ form can be converted to the hydrogen (H-form) by known techniques, such as calcination at high temperature. The Na form can be converted to the hydrogen form (H-form) by converting first to an $NH_4$ form by ion exchange with ammonium salts such as ammonium nitrate. Alternatively, the MOR zeolite may be synthesised using known techniques. The preparation of mordenite from aqueous inorganic compositions is well known and there are several recipes in the literature for making mordenite such as the method described in US 4,205,052.

Suitably, the silica : alumina molar ratio of the starting MOR zeolite which is to be subsequently dealuminated is at least 10, typically in the range 10 to 90 : 1, for example 10 to 40: 1.

One method by which dealumination of the MOR type zeolite may be achieved is to treat the MOR zeolite with a hexafluorosilicate salt. It has been found that treatment of a MOR zeolite with a hexafluorosilicate salt allows aluminium to be removed from the 12-membered ring channels of the zeolite and also from the surface of the zeolite.

Thus, in one aspect of the present invention, there is provided a process for the production of at least one of acetic acid and methyl acetate which process comprises the carbonylation of at least one carbonylatable reactant selected from methanol, dimethyl ether and dimethyl carbonate with carbon monoxide in the presence of a zeolite catalyst of structure type MOR, wherein said zeolite has been dealuminated by treatment with a hexafluorosilicate salt.

The MOR zeolite to be treated with the hexafluorosilicate salt may be, for example, the hydrogen form or ammonium form of the MOR zeolite.

Suitable hexafluorosilicate salts include alkali metal hexafluorosilicates, such as lithium, sodium and potassium hexafluorosilicates. Preferably, however, ammonium hexafluorosilicate is used.

Depending on the particular hexafluorosilicate chosen, the hexafluorosilicate may be used in solid form or as an aqueous solution.

The alkali metal hexafluorosilicates are water soluble and therefore may be used to dealuminate MOR zeolites in solution form. However, due to their involatility, the alkali metal hexafluorosilicates are generally not suitable for use in the solid form.

Ammonium hexafluorosilicate may be used as an aqueous solution or as a solid.

Where solid ammonium hexafluorosilicate is employed, the MOR zeolite and ammonium hexafluorosilicate are mixed, generally at a weight ratio of ammonium hexafluorosilicate to the MOR zeolite in the range of 0.02 : 1 to 1 : 1, such as 0.02 : 1 to 0.1 : 1. The mixture is then heated, under vacuum or preferably, in the presence of an inert gas, such as argon, nitrogen, helium and the like. The heating is preferably carried out at a temperature not exceeding 190 °C to avoid decomposition of the ammonium hexafluorosilicate but which temperature is sufficient to cause the hexafluorosilicate to volatilise. Suitably, the heating is carried out at a temperature in the range 150 to 180 °C. The heating may be carried out for as long as required to provide the desired dealumination. Thereafter the treated material is washed with water, preferably deionised water. The amount of water used for the washing step is preferably sufficient to remove fluorinated impurities from the pores of the zeolite, predominantly, $NH_4AlF_4$ and non-sublimed $NH_4HF_2$. The presence of fluorinated impurities in the washed zeolite may be determined by X-ray diffraction (XRD) or $^{19}$F MAS NMR spectroscopy. Optionally, a further washing with hot water, such as boiling water (100 °C), may be carried out to remove any remaining fluorinated impurities. The washed material is then dried, for example, in air or under vacuum. Drying may be carried out at a temperature in the range 80 to 120 °C.

Alternatively, dealumination of the MOR zeolite may be carried out by contacting the MOR zeolite with an aqueous solution of an alkali metal or ammonium hexafluorosilicate. Suitable procedures are described, for example, in Garralon et al. Zeolites 8 (1988) 268. Typically, the contacting is accomplished by placing an ammonium exchanged MOR zeolite into an aqueous reaction medium, such as an aqueous solution of ammonium acetate, and slowly adding an aqueous solution of ammonium hexafluorosilicate. After allowing the reaction to proceed, a MOR zeolite having an increased silica to alumina ratio is produced. The magnitude of the increase is dependent at least in part on the amount of the ammonium fluorosilicate solution contacted with the zeolite and on the reaction time allowed. Normally a reaction time of between 1 to 5 hours is sufficient for equilibrium to be achieved. It has been found that reaction times longer than 5 hours may result in decreased crystallinity of the MOR zeolite and that reaction times of less than 1 hour may result in only partial reaction of the ammonium hexafluorosilicate. The resulting solid product may be separated from the aqueous reaction medium by conventional filtration techniques.

Treatment of the MOR zeolite with the hexafluorosilicate results in the removal of aluminium atoms from the 12-membered ring channels and/or surface aluminium, thereby increasing the silica : alumina ratio of the MOR zeolite. The extent to which aluminium is removed by treatment with the hexafluorosilicate in either aqueous or solid form will depend on the duration of the reaction, the reaction temperature and the relative concentrations of the zeolite and the hexafluorosilicate.

If desired, increased dealumination may be achieved by repeating the treatment with the hexafluorosilicate, one or more times.

The dealuminated MOR zeolites obtained by treatment with the hexafluorosilicate have increased silica : alumina ratios from the starting materials. Generally, the silica : alumina ratio of the dealuminated zeolite is increased by an amount in the range 5 to 100%.

The silica : alumina molar ratio of the dealuminated MOR zeolite may be determined by the analytical techniques, aluminium ICP (inductively coupled plasma), XRF (X-ray fluorescence) or $^{29}$Si MAS NMR spectroscopy.

For use in the carbonylation process of the present invention, the dealuminated MOR zeolite may be employed in the hydrogen form (H-form) or it may be optionally ion-exchanged or otherwise loaded with one or more metals such as copper, silver, gold, nickel, iridium, rhodium, platinum, palladium or cobalt.

Where the dealuminated MOR zeolite is in the ammonium form it may be converted to the hydrogen form by calcination.

The metal loading on the MOR zeolite may be expressed in terms of the fractional loading of the metal as gram atoms of metal per gram atom of aluminium in the zeolite. The metal loading can also be expressed as a mole percentage loading relative to aluminium in the zeolite through the relationship :

$$\text{mol\% Metal} = (\text{gram atoms Metal/gram atoms aluminium}) \times 100$$

Thus, for example, a loading of 0.55 gram atoms of copper per aluminium in the zeolite equates to a 55 mol% loading of copper relative to aluminium in the zeolite.

Suitably, the metal loading may be in the range of 1 to 200 mol% relative to aluminium in the zeolite.

The process of the present invention employs at least one of methanol, dimethyl ether and dimethyl carbonate as the carbonylatable reactant.

Where dimethyl ether or dimethyl carbonate is used as the carbonylatable reactant, the carbonylation process is typically carried by passing the dimethyl ether or dimethyl carbonate, carbon monoxide and optionally hydrogen, through a fixed or fluidised bed of MOR zeolite catalyst, such as mordenite, maintained at the required temperature, such as in the range 150 to 350 °C, for example, in the range 250 to 350 °C.

Where dimethyl ether or dimethyl carbonate is used as the carbonylatable reactant the process is typically carried out under substantially anhydrous conditions, i.e. in the substantial absence of water. The carbonylation of dimethyl ether or dimethyl carbonate to methyl acetate does not generate water in-situ. Water has been found to inhibit the carbonylation of dimethyl ether or dimethyl carbonate to form methyl acetate. Thus, where dimethyl ether or dimethyl carbonate is the carbonylatable reactant in the process of the present invention, water is kept as low as is feasible. To accomplish this, the dimethyl ether, dimethyl carbonate and carbon monoxide reactants (and catalyst) are preferably dried prior to introduction into the process. However, small amounts of water may be tolerated without adversely affecting the formation of methyl acetate. Suitably, the dimethyl ether or dimethyl carbonate feed may contain up to 2.5 wt% water.

Where dimethyl ether or dimethyl carbonate is the carbonylatable reactant, the primary product of the process is methyl acetate but small amounts of acetic acid may also be produced. The methyl acetate produced by the process of the present invention can be removed in the form of a vapour and thereafter condensed to a liquid.

The methyl acetate may be recovered and sold as such or it may be forwarded to other chemical processes. Where the methyl acetate is recovered from the carbonylation reaction products, some or all of it may be hydrolysed to form acetic acid. Alternatively, the entire carbonylation reaction product may be passed to a hydrolysis stage and acetic acid separated thereafter. The hydrolysis may be carried out by known techniques such as reactive distillation in the presence of an acid catalyst.

Where the carbonylatable reactant is methanol, the carbonylation process is typically carried by passing the methanol, carbon monoxide and optionally hydrogen, through a fixed or fluidised bed of MOR zeolite catalyst, such as mordenite maintained at the required temperature, such as in the range 250 to 400 °C, for example, 275 to 350 °C.

Where the carbonylatable reactant is methanol, the dominant product will be acetic acid, but some methyl acetate may be present, depending on the degree of conversion of methanol.

The carbon monoxide may be substantially pure carbon monoxide, for example, carbon monoxide typically provided by suppliers of industrial gases, or it may contain impurities that do not interfere with the conversion of the dimethyl ether to methyl acetate, such as nitrogen, helium, argon, methane and/or carbon dioxide.

The process of the present invention may be carried out in the presence of hydrogen. The hydrogen may be fed as a separate stream to the carbonylation reactor or it may be fed in combination with, for example carbon monoxide. Mixtures of hydrogen and carbon monoxide are commercially produced by the steam reforming of hydrocarbons and by the partial oxidation of hydrocarbons. Such mixtures are commonly referred to as synthesis gas. Synthesis gas comprises mainly carbon monoxide and hydrogen but may also contain smaller quantities of carbon dioxide.

Suitably, the molar ratio of carbon monoxide : hydrogen may be in the range 1 : 3 to 15 : 1, such as 1 : 1 to 10 : 1, for example, 1 : 1 to 4 : 1.

The molar ratio of carbon monoxide to carbonylatable reactant is suitably in the range 1 : 1 to 99 : 1, such as 2 : 1 to 60 : 1.

Where the process is to be conducted substantially in the absence of water, it is preferred that the MOR zeolite catalyst is dried prior to use. The zeolite may be dried, for example by heating to a temperature of 400 to 500 °C.

It is preferred that the zeolite catalyst is activated immediately before use by heating the zeolite at elevated temperature for at least one hour under flowing nitrogen, carbon monoxide, hydrogen or mixtures thereof.

The process of the present invention may be carried out at a total pressure in the range 1 to 100 barg. Suitably, the pressure may be in the range of 10 to 80 barg.

Where hydrogen is employed, the hydrogen partial pressure is suitably in the range 0.1 to 50 barg.

The carbon monoxide partial pressure should be sufficient to permit the production of the carbonylation reaction product, but is suitably in the range 0.1 to 50 barg.

The Gas Hourly Space Velocity (GHSV) is suitably in the range 500 to 40,000 h$^{-1}$, such as 2000 to 20,000 h$^{-1}$.

Preferably, the process of the present invention is carried out substantially in the absence of halides, such as iodide. By the term 'substantially' is meant that the halide, for example, iodide content of the reactant gases (dimethyl ether and carbon dioxide) and catalyst is less than 500 ppm, preferably less than 100 ppm.

The process may be operated as either a continuous or a batch process, preferably as a continuous process.

The invention is now illustrated with reference to the following Examples.

## Example 1

This example illustrates the preparation of a dealuminated H-mordenite carbonylation catalyst using solid ammonium hexafluorosilicate (($NH_4$)$_2SiF_6$)

Catalyst A (comparative) was prepared by calcining a H-mordenite of silica : alumina ratio 20 (ex Zeolyst International) at 500 °C in static air for 3 hours.

Catalyst B was prepared by mixing 45g of H-mordenite of silica : alumina ratio 20 (ex Zeolyst International) with 5.9g of solid ammonium hexafluorosilicate (ex Johnson Matthey plc. The mixture was transferred to a reactor and fluidised with argon. The mixture was then heated at 180 °C for 5 hours in an oven under a continuous stream of argon, followed by washing with 4 litres of deionised water at room temperature and then with 4 litres of boiling water at (100 °C). The washed mordenite was dried overnight at a temperature of 100 °C. 40.6g of dealuminated H-mordenite was obtained. The silica : alumina ratio of the dealuminated H-mordenite was analysed by XRF and $^{29}$Si MAS NMR and determined to be 21.6.

## Example 2

This example illustrates the use of each of the Catalysts A and B for the carbonylation of dimethyl carbonate.

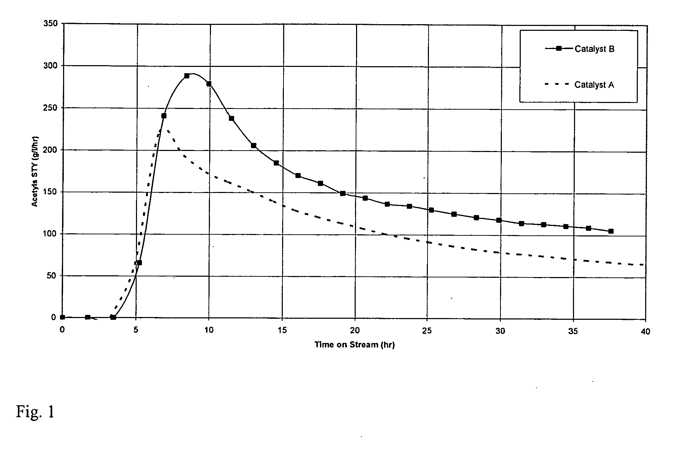

A Hastelloy reactor tube was packed with 0.6 ml of a catalyst and 0.2g of a gamma alumina pre-bed. Prior to use, each catalyst had been pressed (32mm die in a Specac press at 12 tonnes) and sieved to a particle size in the range 250 to 500 microns. The portion of the reactor tube containing the catalyst was heated by means of an electrical heating jacket. The reactor and heating jacket were themselves mounted in a heated cabinet which maintained the temperature of the pre-bed. The reactor was heated at atmospheric pressure under a flow of nitrogen to 130 °C in the heated cabinet. Once at temperature, the feed gas was changed to 80 mole % carbon monoxide and 20 mole % hydrogen and the reactor system was pressurised to 20 barg. The gas mixture was fed to the reactor from a gas header through a mass flow controller at a gas flow rate of 5000 per hour. The reactor was heated to 300°C at a ramp rate of 3°C per minute using the electrical heating jacket. Once at temperature these conditions are maintained for two hours, after which the carbonylation reaction was started by introducing dimethyl carbonate at a flow rate sufficient to achieve a gas feed comprising 76 mole % carbon monoxide, 19 mole % hydrogen and 5 mole % dimethyl carbonate. A constant flow of reaction off-gases was taken from the high pressure side of the reactor system through a needle valve, let down to atmospheric pressure while maintaining a temperature of at least 130 °C and passed to a gas chromatograph for analysis. From the gas chromatography analysis of the reactor effluent for methyl acetate and acetic acid the space time yield (STY) of acetyls products was calculated as the molar equivalent weight of acetic acid corresponding to the sum of the methyl acetate and acetic acid produced expressed as grams of acetic acid per hour per litre of catalyst. Fig. 1 depicts the space time yield to acetyls products (g/l/h) versus time on stream (hours) for the carbonylation of dimethyl carbonate using Catalysts A and B.

The data in Fig. 1 clearly shows that dealuminated Catalyst B is more effective than the non-dealuminated Catalyst A in converting dimethyl carbonate to the carbonylation products, methyl acetate and acetic acid.

## Example 3

This example illustrates the preparation of dealuminated H-mordenite carbonylation catalyst using an aqueous solution of ammonium hexafluorosilicate (($NH_4$)$_2SiF_6$).

Catalyst F was prepared by treating 267.8g of mordenite in the ammonium form (ex Zeolyst International) of silica : alumina ratio of 20 with a 3 molar solution of ammonium acetate at an ammonium acetate : mordenite ratio of 3 (wt/wt). The solution was heated to a temperature of 75 °C under reflux at which point a 0.4 molar solution of ammonium hexafluorosilicate was added at a rate of 12ml/h. The amount of ammonium hexafluorosilicate added was such that the ratio of ammonium hexafluorosilicate :

mordenite was 0.1 (wt/wt). On completion of the ammonium hexafluorosilicate addition, the solution is heated to 95 °C and stirred for a period of time sufficient to take the total period of time from the start of the ammonium hexafluorosilicate addition to 3 hours. The solution was then filtered to recover the solid dealuminated $NH_4$-mordenite. The recovered solid was washed 5 times with boiling water, in an amount to give a water : mordenite ratio of 200 (wt/wt). The washed dealuminated $NH_4$-mordenite was then calcined at 500 °C for 3 hours in air to obtain a dealuminated H-mordenite. The silica : alumina ratio of this material was determined to be 24.2 from ICP analysis.

### Example 4

This example illustrates the preparation of a non-dealuminated copper-exchanged mordenite carbonylation catalyst (Catalyst G).

Cu-mordenite was prepared by a two step ion exchange method. In the first step, 9.58 g of a mordenite (in its ammonium form) with a silica : alumina ratio of 20 (ex Zeolyst International.) was mixed with 100 ml of an aqueous solution of copper nitrate (1.507 g of $Cu(NO_3)_2\cdot3H_2O$) and refluxed at 80 °C for 2 hours to obtain a slurry. The slurry was filtered and dried at 100 °C for 12 hours to obtain a solid. The solid was mixed with 87 ml of a copper nitrate solution (0.78 g of $Cu(NO_3)_2.3H_2O$) and refluxed at 80 °C for 2 hours, filtered and then dried at 100 °C for 12 hours. After being dried, the solid was calcined at 500°C in air for two hours.

### Example 5

This example illustrates the preparation of a non-dealuminated H-mordenite carbonylation catalyst (Catalyst H).

Ammonium mordenite having a silica : alumina ratio of 20 (ex Zeolyst International) was dried at 100 °C for 12 hours and then calcined at 500°C in air for two hours.

### Example 6

This example illustrates the use of the catalysts F-H, prepared in Examples 3-5, for the carbonylation of methanol.

The carbonylation reactions were performed in a down-flow stainless steel fixed bed reactor with a 10 mm internal diameter. 1 g of a catalyst F-G (sieved between 0.25 mm and 0.42 mm particle size) was loaded into the reactor after dilution with SiC (0.64 mm-0.25 mm) to obtain a bed volume of 6.4 cc. Carbon monoxide and methanol were fed to the reactor by means of mass-flow controllers. In order to ensure the homogeneity of the reactant mixture, methanol was vaporized and mixed with the carbon monoxide in a preheater maintained at 150 °C. During the reaction, the pressure was controlled electronically through a Badger pneumatic valve.

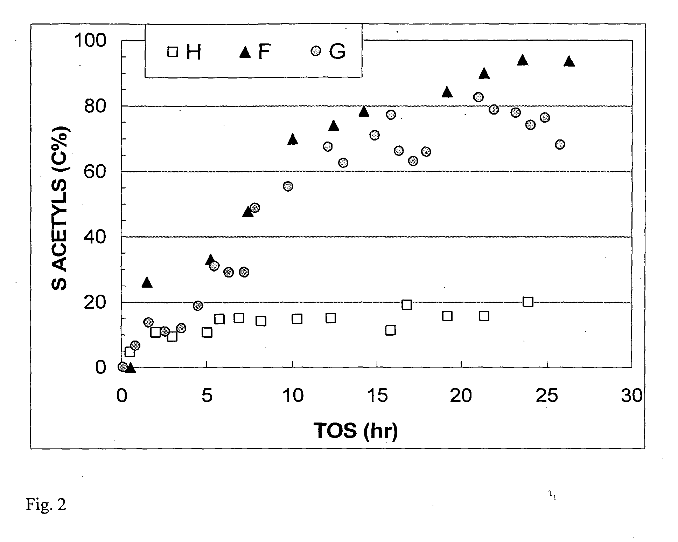

Prior to use, the catalyst was activated under flowing argon (50 cc/$g_{cat}$·h) at 350 °C (ramp rate of 2 °C/min) for 16 hours followed by contacting the catalyst with carbon monoxide (100 cc/$g_{cat}$·h) for 2 hours at 350°C. After the activation treatment, the reaction pressure was increased to 10 bar with carbon monoxide. Carbon monoxide, argon and methanol at a molar ratio of methanol : carbon monoxide : argon of 1 : 10 : 1 were fed to the reactor at a gas hourly space velocity of 3000 h$^{-1}$. The exit stream from the reactor was analysed periodically for carbonylation reaction products and unreacted reactants by on-line gas chromatography using a Varian 3800 chromatograph equipped with three columns and two detectors. Analysis of argon, carbon monoxide, carbon dioxide and methane was performed using two packed columns, a Porapak Q (0.5 m length) and a 13X molecular sieve (1.5 m length), and a thermal conductivity detector (TCD). Oxygenates (including acetyls) and hydrocarbons were analyzed using a capillary column (WCOT fused silica, 2.5 m length) and a flame ionization detector (FID). Fig. 2 depicts the selectivity to acetyls products (acetic acid and methyl acetate) obtained versus time on stream for the carbonylation of methanol using catalysts F to G. %selectivity to acetyls products was calculated as ( moles methyl acetate + moles acetic acid)/total moles of carbon containing products.

The data in Fig. 2 illustrates that the dealuminated H-mordenite catalyst (catalyst F) is considerably more selective to acetyls products than the non-dealuminated H-mordenite (Catalyst H). It is also more selective than the non-dealuminated copper promoted mordenite catalyst, Catalyst G.

### Example 7

This example illustrates the preparation of a non-dealuminated mordenite catalyst (Catalyst I), and a dealuminated H-mordenite (Catalyst K).

**Preparation of Catalyst I**

Ammonium mordenite of silica : alumina ratio of 20 (ex Zeolyst International) was placed in a calcination furnace and was heated to 450 °C using the following temperature ramping program: temperature ramped from ambient to 90 °C over 30 min, held at 90 °C for 120 min, heated to 110 °C over 30 min, held at 110 °C for 120 min, heated to 450 °C over 60 minutes and then held at that temperature for 3 hours. The H-mordenite catalyst was then allowed to cool to ambient temperature.

**Preparation of Catalyst K**

A dealuminated H-mordenite (Catalyst K) was prepared according to the method used to prepare Catalyst F in Example 4 and then calcined using the calcination method described in the preparation of Catalyst I above.

**Example 8**

This example illustrates the use of catalysts I and K, prepared in Example 7, for the carbonylation of dimethyl carbonate.
Carbonylation reactions using catalysts I and K were carried out using the procedure described in Example 2 above.

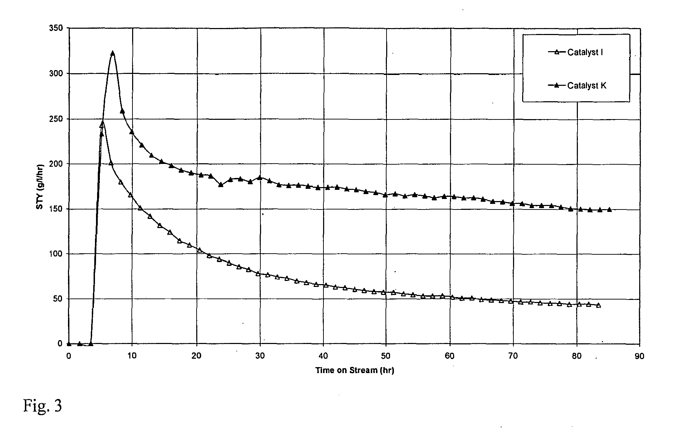

Fig. 3 depicts the STY to acetyls products (g/l/h) versus time on stream (hours) for the carbonylation of dimethyl ether using catalysts I and K.
As can be seen from Fig. 3 the STY to acetyls products (acetic acid and methyl acetate) using the dealuminated catalyst K is approximately double that of the STY obtained using the non-dealuminated catalyst I.

**Claims**

1.  A process for the production of at least one of acetic acid and methyl acetate which process comprises the carbonylation of at least one carbonylatable reactant selected from methanol, dimethyl ether and dimethyl carbonate with carbon monoxide in the presence of a zeolite catalyst of structure type MOR, wherein said zeolite is dealuminated.

2.  A process according to claim 1 wherein the zeolite is dealuminated by treatment with a hexafluorosilicate salt.

3.  A process according to claim 2 wherein the hexafluorosilicate salt is an alkali metal hexafluorosilicate salt.

4.  A process according to claim 2 wherein the hexafluorosilicate salt is ammonium hexaflurosilicate.

5.  A process according to claim 3 wherein the alkali metal hexafluorosilicate salt is used as an aqueous solution.

6.  A process according to claim 4 wherein the ammonium hexaflurosilicate is used as an aqueous solution or as a solid.

7.  A process according to any one of claims 1 to 6 wherein the MOR zeolite to be dealuminated is in the hydrogen form or ammonium form of the zeolite.

8.  A process according to any one of claims 1 to 7 wherein the dealuminated zeolite is in the ammonium form and converted to the hydrogen form by a calcination process.

9.  A process according to any one of claims 1 to 8 wherein, for use in the carbonylation, the dealuminated MOR zeolite is used in the hydrogen form or ion-exchanged or otherwise loaded with at least one metal selected from copper, silver, gold, nickel, iridium, rhodium, platinum, palladium and cobalt.

10.  A process according to any one of claims 1 to 9 wherein the carbonylatable reactant is methanol.

11.  A process according to any one of claims 1 to 9 wherein the carbonylatable reactant is selected from at least one of dimethyl ether and dimethyl carbonate.

12.  A process according to claim 11 wherein the process is carried out under substantially anhydrous conditions.

**13.** A process according to any one of claims 1 to 12 wherein the process is carried out in the presence of hydrogen.

Fig. 1

Fig. 2

Fig. 3

Europäisches
Patentamt

European
Patent Office

Office européen
des brevets

## EUROPEAN SEARCH REPORT

Application Number

EP 08 25 3322

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEUNG P ET AL: "Site requirements and elementary steps in dimethyl ether carbonylation catalyzed by acidic zeolites" JOURNAL OF CATALYSIS, ACADEMIC PRESS, DULUTH, MN, US, vol. 245, no. 1, 1 January 2006 (2006-01-01), pages 110-123, XP002453503 ISSN: 0021-9517 Second paragraph in section 2.1; scheme 1; fig. 12 ----- | 1-13 | INV. C07C51/12 C07C67/36 C07C67/37 |
| X | US 2007/238897 A1 (CHEUNG PATRICIA [US] ET AL CHEUNG PATRICIA [US] ET AL) 11 October 2007 (2007-10-11) * paragraphs [0038], [0059], [0060], [0065]; table 3 * ----- | 1-13 | |

TECHNICAL FIELDS
SEARCHED      (IPC)

C07C

The present search report has been drawn up for all claims

| Place of search | Date of completion of the search | Examiner |
|---|---|---|
| Munich | 2 March 2009 | Cooper, Simon |

EPO FORM 1503 03.82 (P04C01)

## ANNEX TO THE EUROPEAN SEARCH REPORT
## ON EUROPEAN PATENT APPLICATION NO.

EP 08 25 3322

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2009

| Patent document cited in search report | Publication date | Patent family member(s) | Publication date |
|---|---|---|---|
| US 2007238897 A1 | 11-10-2007 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0596632 A
- WO 2006121778 A
- WO 2005105720 A
- US 3551353 A
- US 4588496 A
- US 5411724 A
- US 4205052 A

**Non-patent literature cited in the description**

- **W M Meier ; D H Olson ; Ch Baerlocher.** Atlas of Zeolite Structure Types. Elsevier, 2007